(19) **Europäisches Patentamt**

**European Patent Office**

**Office européen des brevets**

(11) **EP 1 407 014 B1**

(12) **EUROPEAN PATENT SPECIFICATION**

(45) Date of publication and mention
of the grant of the patent:
**15.11.2006 Bulletin 2006/46**

(51) Int Cl.:
*C12N 15/10* (2006.01)   *C12N 15/90* (2006.01)
*C12N 15/31* (2006.01)   *C07K 14/255* (2006.01)
*C12N 1/21* (2006.01)   *C07K 16/12* (2006.01)
*C12Q 1/68* (2006.01)   *G01N 33/68* (2006.01)
*A61K 31/711* (2006.01)   *A61K 39/112* (2006.01)
*A61K 39/395* (2006.01)

(21) Application number: **02743053.7**

(22) Date of filing: **17.05.2002**

(86) International application number:
**PCT/EP2002/005493**

(87) International publication number:
**WO 2002/092814 (21.11.2002 Gazette 2002/47)**

(54) **SCREENING METHOD FOR ATTENUATING OR VIRULENCE DEFECTIVE MICROBIAL CELLS**

METHODE ZUM AUFFINDEN VON ABGESCHWÄCHTEN ODER VIRULENT-DEFEKTEN
MIKROBEN

PROCEDE DE CRIBLAGE POUR L'ATTENUATION OU LA VIRULENCE DES CELLULES
MICROBIENNES DEFECTUEUSES

(84) Designated Contracting States:
**AT BE CH CY DE DK ES FI FR GB GR IE IT LI LU
MC NL PT SE TR**

(30) Priority: **17.05.2001 EP 01112158**
**23.07.2001 EP 01117866**

(43) Date of publication of application:
**14.04.2004 Bulletin 2004/16**

(73) Proprietor: **Creatogen Aktiengesellschaft**
**86156 Augsburg (DE)**

(72) Inventors:
• **FREISSLER, Elke**
**89075 Ulm (DE)**
• **FUCHS, Thilo, M.**
**86161 Augsburg (DE)**
• **NIESALLA, Heide, S.**
**86153 Augsburg (DE)**
• **APFEL, Heiko**
**86356 Neusäss (DE)**

(74) Representative: **Weiss, Wolfgang et al**
**Weickmann & Weickmann**
**Patentanwälte**
**Postfach 86 08 20**
**81635 München (DE)**

(56) References cited:
WO-A-00/14240      WO-A-00/73336
WO-A-00/73502      WO-A-01/02555
WO-A-86/01829      WO-A-96/17951
WO-A-99/37330      WO-A-99/50402
WO-A-02/083940     US-A- 5 434 065

• FIELDS P I ET AL: "MUTANTS OF SALMONELLA-TYPHIMURIUM THAT CANNOT SURVIVE WITHIN THE MACROPHAGE ARE AVIRULENT" PROCEEDINGS OF THE NATIONAL ACADEMY OF SCIENCES OF THE UNITED STATES, vol. 83, no. 14, 1986, pages 5189-5193, XP002241678 1986 ISSN: 0027-8424 cited in the application

• HENSEL M ET AL: "WHOLE GENOME SCAN FOR HABITAT-SPECIFIC GENES BY SIGNATURE-TAGGED MUTAGENESIS" ELECTROPHORESIS, WEINHEIM, DE, vol. 19, no. 4, April 1998 (1998-04), pages 608-612, XP001085303 ISSN: 0173-0835

• POLISSI ALESSANDRA ET AL: "Large-scale identification of virulence genes from Streptococcus pneumoniae" INFECTION AND IMMUNITY, AMERICAN SOCIETY FOR MICROBIOLOGY. WASHINGTON, US, vol. 66, no. 12, December 1998 (1998-12), pages 5620-5629, XP002136502 ISSN: 0019-9567 cited in the application

EP 1 407 014 B1

- CLAUS H ET AL: "Identification of a hotspot for transformation of Neisseria meningitidis by shuttle mutagenesis using signature-tagged transposons" MOLECULAR AND GENERAL GENETICS, SPRINGER VERLAG, BERLIN, DE, vol. 259, no. 4, 1998, pages 363-371, XP002175425 ISSN: 0026-8925 cited in the application
- MAHAN M J ET AL: "SELECTION OF BACTERIAL VIRULENCE GENES THAT ARE SPECIFICALLY INDUCED IN HOST TISSUES" SCIENCE, AMERICAN ASSOCIATION FOR THE ADVANCEMENT OF SCIENCE,, US, vol. 259, 29 January 1993 (1993-01-29), pages 686-688, XP001026153 ISSN: 0036-8075 cited in the application
- LEE M.S.; SEOK C.; MORRISON D.A.: 'Insertion-duplication mutagenesis in Streptococcus pneumoniae: targeting fragment length is a critical parameter in use as a random insertion tool' APPLIED AND ENVIRONMENTAL MICROBIOLOGY vol. 64, no. 12, December 1998, WASHINGTON, DC, US, pages 4796 - 4802, XP002254569
- CHALKER A.F. ET AL: 'Systematic identification of selective essential genes in Helicobacter pylori by genome prioritization and allelic replacement mutagenesis' JOURNAL OF BACTERIOOGY vol. 183, no. 4, February 2001, WASHINGTON, DC, US, pages 1259 - 1268, XP002245013

**Description**

This invention relates to a novel method for the genome saturating identification of nucleic acid sequences which are essential for infectivity and/or intracellular survival and/or propagation of microbial sequences in permissive eukaryotic host cells. Further, a method for the identification of attenuated microorganisms and novel antimicrobial compounds using the identified essential nucleic acids and proteins encoded thereby is provided.

Infection processes have been demonstrated to be coordinately regulated or stimulated by host factors encountered *in vivo,* and these were found to be multifactoral and dynamic. Infection and infectivity relate to the ability of microbial cells to be taken up by eukaryotic host cells or to be internalized leading to a possible intracellular propagation of the microbial cells and subsequent reaction of the host. An understanding of infection and the definition of virulence evolved as one became aware that the pathogenic potential of microorganisms could not be explained by the sole contribution of one or a few so-called virulence determinants. Virulence relates to the property of microbial cells to infect eukaryotic cells. This concept of virulence was continuously redefined by innovative approaches designed to identify and characterize facultatively essential genes, whereby several genes were found to be environmentally regulated in many pathogenic microbial cells. Facultatively essential genes are nucleic acid sequences which are essential for infectivity and/or intracellular survival and/or propagation in permissive eukaryotic host cells.

Recent advances in the field of bacterial pathogenesis have allowed a large-scale identification of new virulence genes in different bacterial species. The methods developed so far are based on the concept that specific gene products are required for each stage of an infection process and that their expression is often regulated by the different environmental conditions encountered in the host.

With a better understanding of infection processes and the discovery of genes which play an important role therein the development of new antimicrobial drugs, such as attenuated microorganisms used as live vaccines or new compounds, comes into prospect. An attenuated microorganism has a reduced capacity to infect and/or survive and/or propagate within permissive eukaryotic host cells compared to a wild-type organism due to a mutation in a facultatively essential gene and is thereby suitable as live vaccine or as carrier for heterologous antigens.

Accordingly, a need for a novel method for reliably identifying facultatively essential nucleic acid sequences which are necessary for infectivity, intracellular survival or propagation in the host in a large scale exists.

Several approaches have been described so far to identify those genes. These include:

**1. DNA Microarray technology [Schena et al. 1995 Science 467-470]**

The unifying principle of all microarray experiments is that labeled nucleic acid molecules in solution hybridize to complementary sequences immobilized on a solid substrate, thus facilitating parallel quantitative measurement of different sequences in a complex mixture.

Exploration of pathogen gene expression in the host environment is technically challenging because of the relatively small number of pathogens present in an infected animal. However, as it is demanded for candidate genes identified by any expression screening approach, a role in pathogenesis must be confirmed by mutation and virulence assays which is time consuming and laborious. One further important caveat of studying gene transcription in a system is that post-transcriptional regulatory events cannot be detected.

**2. Differential fluorescence induction (DFI)**

The method is based on a genetic selection via fluorescence-enhanced GFP and a fluorescence activated cell sorter (FACS) to allow identification of virulence genes expressed by pathogens that are differentially expressed when a bacterium associates with its host cell [Valdivia 1996, Valdivia 1997]. Random DNA fragments are inserted into a promoterless gfpmut plasmid, creating gene fusions. The enrichment strategy is based on the ability to separate bacteria by FACS in response to the expression, or lack of expression, of the fluorescent marker.

However, several rounds of FACS analysis are necessary to exclude false positives and negatives, therefore reducing the number of potential candidates. Minimizing the false positives and negatives in several rounds of testing means, that the potential candidates are laborious to obtain.

Due to this selection strategy, only virulence genes that are differentially expesed in the selected environments, will be identified. Genes that are constantly expressed and concomitant facultatively essential are not found by this method as they have the same expression status all the time. Therefore, this method will not cover the whole set of facultatively essential genes in a pathogen.

**3. *In vivo* expression technology (IVET) [Mahan et al. 1993 Science 259:686-688]**

The IVET system allows the study of the bacterial response to the host environment in situ using a gene expression scheme within the animal host itself for selection of genes that are specifically expressed during the infection. The method is based on complementation of a promoterless synthetic operon with an attenuating auxotrophic mutation fused to chromosomal DNA fragments. In conditions prevailing *in vivo,* wild-type recombinant strains having no functional promoters are not viable. Fusions containing a promoter, either constitutive or induced *in vivo,* allow transcription and bacterial survival. Identification of promoters which are optimally expressed in animal tissues provides a means of establishing *in vivo* regulated expression of heterologous antigens in live vaccines. The elucidation of these gene products should provide new targets for antimicrobial drug development.

However, IVET is limited in the isolation of genes dependent upon transcriptional regulation. Genes activated or regulated posttranscriptionally could not be isolated so far. Another drawback of this approach is, that IVET has to be used with care in heterologous bacterial systems because transcription and translation signals of recombinant fusions may not be recognized. Therefore, not the whole set of facultatively essential genes can be be detected.

Besides, the method depends on the construction of transcriptional fusions of promoter sequences and the use of a well-characterized mutant host strain as an auxotrophic bacterial strain is necessary. The system can also be used with a selection based on antibiotic resistance in the host rather than on nutritional deficiencies. However, this can be problematical in systemic distribution of e.g. chloramphenicol and present limitations for selection of chloramphenicol resistance in certain animal models [Mahan et al 1995].

**4. Signature Tagged Mutagenesis (STM)**

The method is based on insertional mutagenesis by transposons. Clones with an insertion genotype are generated in vitro and selected by cell culture or an animal model to identify genes essential during infection [Hensel et al 1995, Science 269; 400-403]. However, high frequency of random transposon insertions into the chromosome is not always possible and transposons are not easily utilized in all bacterial species. Modifications for insertion-duplication mutagenesis [Polissi a et al. 1998; I&I 66:5620-5629] and shuttle mutagenesis [Claus et al. 1998; Mol Gen Genet. 259: 363-371 in some bacterial species had to be made.

Despite, the complexity of the pools cannot be enlarged greatly, as the probability increases that some virulent mutants would not be present in sufficient numbers *in vivo* to produce enough labeled probe for hybridization analysis. In the hybridization step of STM, the quantity of labeled tag for each transposon is inversely proportional to the complexity of the tag pool, so that there is a limit to the pool size above which hybridization signals become too weak to be detected. The limited pool size are 96 unique oligonucleotide sequences that do not cross-hybridize. Besides,it depends on the construction of transcriptional fusions of promoter sequences and the use of a well-characterized mutant host strain. Moreover, only mutants that are not recovered from cell culture or an animal, are gleaned from the experiments, whereas smaller differences of attenuation are not found.

**5. Subtractive Recombination Mutagenesis (SRM)**

The method is based on insertional mutagenesis by homologous recombination (WO00/73502). Clones with an insertion genotype are generated in vitro, pooled and selected by cell culture or in the mouse to identify genes essential for cell growth under *in vivo* conditions. Nucleotide fragments of the pool before and after the selection conditions are generated and subtracted from each other. Fragments of genes essential for cell growth, are missing in the pool after the selection. To verify the gene, nucleotide fragments are cloned and tested again in the respective model.

However, to reliably identify single mutants of the pool several rounds of subtraction have to be performed to enrich favored mutants. Besides, a further cloning step is necessary to verify the genes in the respective mouse or cell culture model.

**6. In vitro assay for intracellular survival [Fields et al 1986]**

The method is based on a library of transposon insertional mutations, which is screened for individual mutants showing a lower level of survival in macrophages than parental wild-type cells. Screening is performed in 96 well plates and intracellular survival mutants are selected by plating cell lysates after infection, showing a decrease in number of viable intracellular bacteria relative to the wild-type.

However, only 115 potential macrophage survival mutants have been found with this method. Thus, the screening seems to be incomplete. This might be due to the problems associated with obtaining a high frequency of random transposon insertions into the chromosome. Additionally, the presence of operon structures poses further problems.

Another disadvantage of this method is that no discrimination between different levels of attenuation is possible. Furthermore, the method requires the use of the antibiotic gentamycin in high concentrations which might result in the identification of false positive facultatively essential mutants.

WO86/01829 relates to the production of bacterial mutants by transposon mutagenesis. Transposon mutagenesis has the disadvantage of the appearance of a linear segment of DNA already contained in a cell at a second site in the DNA molecule of the same cell.

Lee et al. (Applied and Environmental Microbiology, 1998, 64:4796-4802) relates to the examination of the transformation efficacy and targeting specifity of insertion-duplication mutagenesis in Streptococcus pneumoniae. However, this document discloses that the insertion duplication mutagenesis is not as effective compared to transposon mutagenesis.

Chalker et al. (Journal of Bacteriology, 2001, 183:1259-1268) relates to a process for identifying biological functions of single genes by the introduction of targeted mutagenesis. This method, however, is very complex, since for every single gene a set of primers needs to be identified in order to establish an insertion cassette. The amplified gene is then used to transform bacteria. The method of Chalker et al. requires a preselection in order to keep the complexity low.

Taken together, no satisfying screening procedure to identify nucleic acid sequences which are essential for infectivity and/or intracellular survival and/or propagation in permissive eukaryotic host cells is known from state of the art.

Thus, the problem underlying the present invention is to provide an improved method for the identification of facultatively essential microbial nucleic acid sequences.

The solution to the above problem is achieved by the method provided by the invention which relates to the identification of microbial nucleic acid sequences which are essential for infectivity and/or intracellular survival and/or propagation in eukaryotic host cells comprising the steps

   (a) subjecting microbial cells to genome saturating mutagenesis comprising the steps:

   (a1) cloning of a genome-representing fragment library of a microbial organism into a conditionally replicating vector, suitable for insertional duplication mutagenesis,
   (a2) transforming the vector comprising the fragment library of (a1) into the microbial organism of (a1) under permissive conditions, and
   (a3) producing mutant cells by insertional duplication mutagenesis,

   (b) cultivating the mutant cells of step (a) and obtaining a library of viable mutant cells,
   (c) contacting permissive eukaryotic host cells with a single clone of the library of step (b) under conditions, wherein an intracellular uptake of the microbial cells into the eukaryotic host cells can take place,
   (d) removing non-intracellular microbial cells from the eukaryotic host cells of step (c) and
   (e) identifying the microbial cells having a reduced capacity of infectivity and/or intracellular survival and/or propagation in said eukaryotic host cells and
   (f) characterizing the obtained nucleic acid sequences and/or polypeptides encoded thereby associated with said reduced capacity.

The method as described is based on the observation that mutated microbes can be classified into different phenotypes *in vivo:* an obligatory essential phenotype, a facultatively essential phenotype and a facultatively non-essential phenotype. The obligatory essential phenotype relates to a microorganism which is not viable at all after mutagenesis because at least one obligatory essential gene is inactivated. The facultatively essential phenotype is viable, i.e. it can be cultured in vitro under suitable growth conditions, but has a reduced capacity to infect, survive and/or propagate within permissive host cells. This reduced capacity is caused by inactivation or inhibition of at least one facultatively essential nucleic acid sequence. The facultatively non-essential phenotype has substantially the same characteristics as the wild-type and thus cannot be differentiated from the wild-type.

The present invention relates to the identification of mutations in facultatively essential genes. Depending on the specific mutation, a cell with the resulting phenotype may have a reduced ability to infect the host cell. On the other hand, it may be able to infect the host cell but exhibits a reduced intracellular survival, or may be able to infect the host cell and survive within the host cell but is defective in propagation. Moreover, cells with combinations of different mutations may exist. Each of these phenotypes is caused by a mutation in a facultatively essential gene which is necessary for infectivity and/or intracellular survival and/or propagation in eukaryotic host cells.

Facultatively essential genes are especially preferred targets for the development of antimicrobial compounds and/or for the generation of attenuated microorganisms which can be used as live vaccines or carriers for heterologous antigens.

The method of the invention has several advantages for identifying facultatively essential microbial genes. Some of these advantages are as follows:

The method is very fast as results are obtained within two or three days. Further, the method may be carried out as a high throughput screening assay within short time and a minimum of tedious efforts. This enables a biological assessment of a complete bacterial genome in such a short time frame which is unique.

The screening may be performed in cell culture and does not require a complex animal model. Moreover, even cultures of primary cells can be used. Besides, minimization of costs is achieved in cell culture compared to animal facilities.

Further, different attenuating mutations can easily be distinguished leading to a rapid classification of interesting mutations found in the screening process. Attenuated microbial microorganisms may be directly obtained without further ado. Therefore, laborious work is minimized as following tests are unnecessary.

The method can be routinely used with a large quantity of different cell lines, pathogens and cell culture conditions. Therefore a broad range of new attenuating mutations can be obtained.

After identification of facultatively essential genes no further mutations need to be introduced to perform subsequent virulence assays in order to identify the level of attenuation of the respected microorganism *in vivo*.

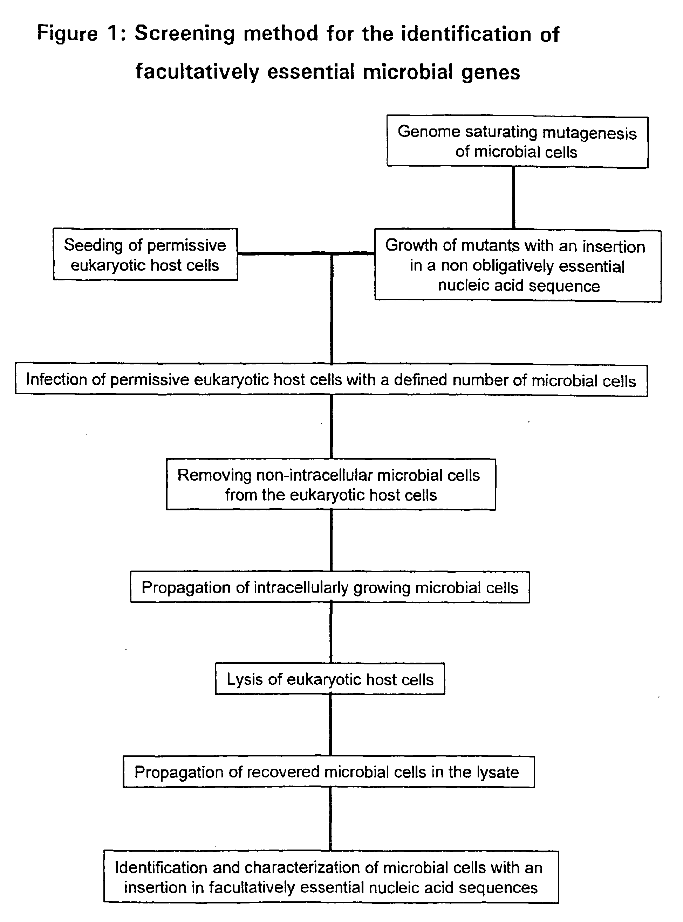

A detailed description of the different steps of the inventive method is as follows (see also Figure 1):

**Step (a) comprises subjecting microbial cells to substantial genome saturating mutagenesis.**

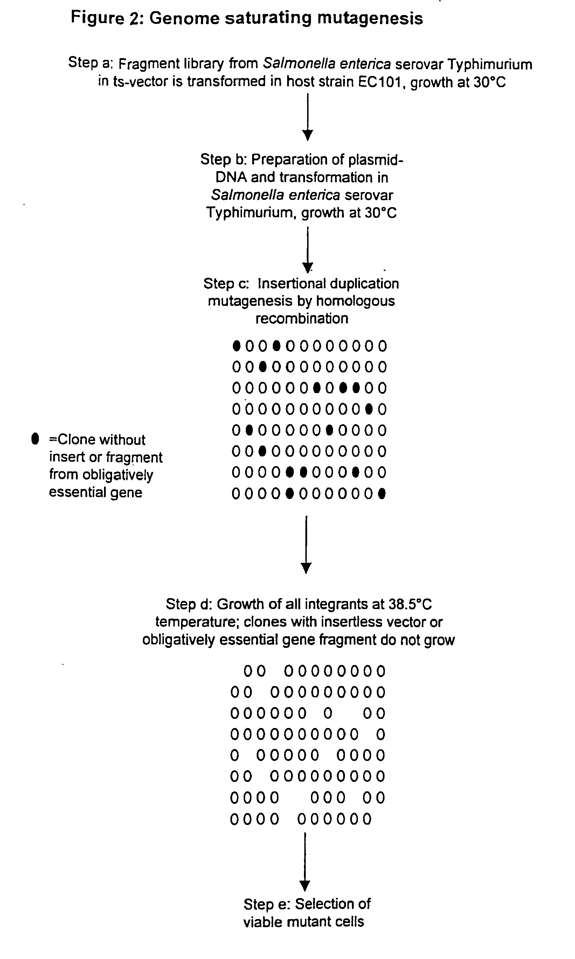

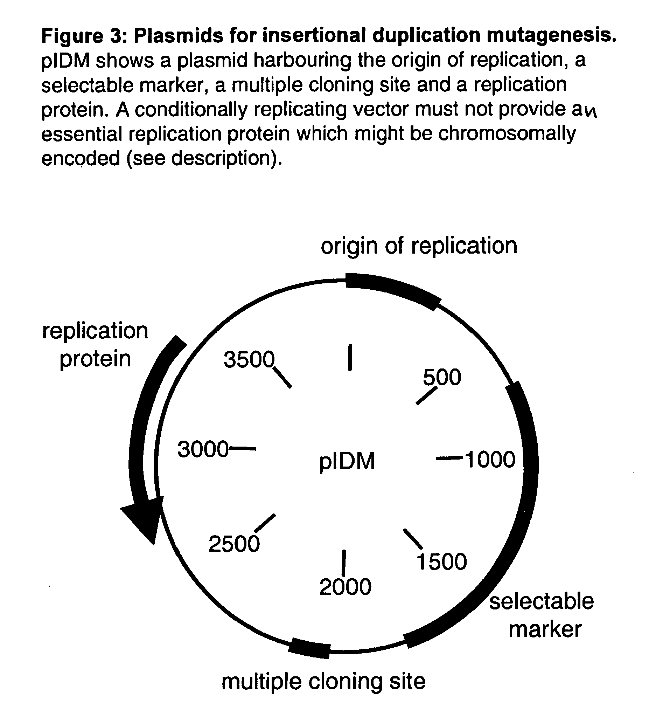

Microbial cells can be mutagenized by any suitable method. However, in order to obtain a library of mutant cells representing the genome of the respective microorganism, a substantial genome saturating mutagenesis procedure is carried out. An appropriate method therefor is described in the copending patent application WO 02/83940. This method is diagrammatically depicted in Figure 2 and comprises the cloning of a genome-representing fragment library of the microorganism of interest into a conditionally replicating vector, i.e. a vector which replicates only under certain permissive conditions, e.g. at a permissive temperature. The vector contains a selection marker gene and should be suitable for insertional duplication mutagenesis (see Figure 3).

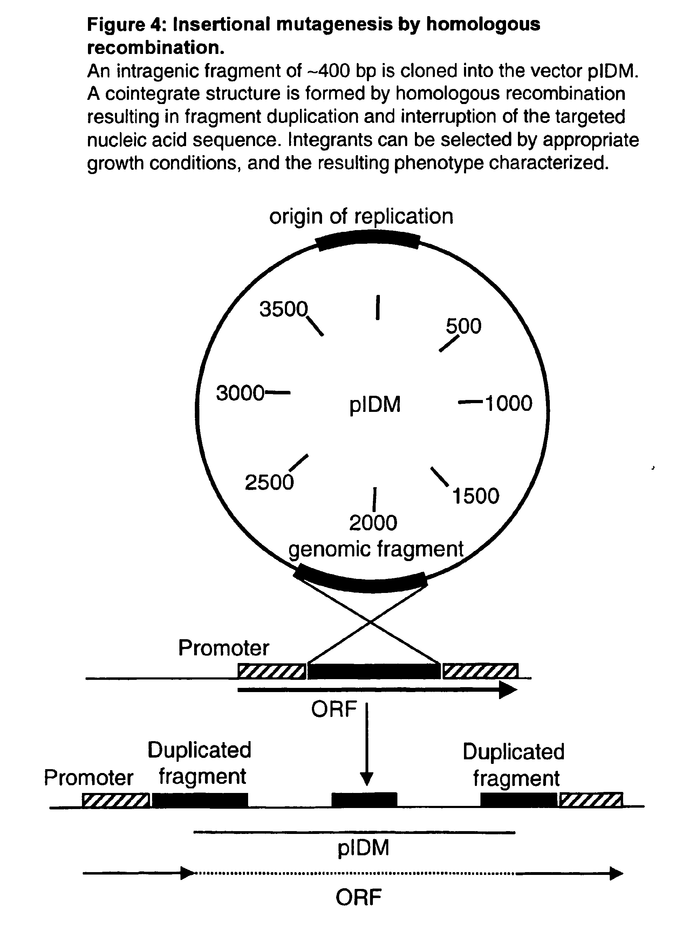

Then the fragment library is transformed into microbial host cells of interest under permissive conditions and insertional mutagenesis takes place by homologous recombination as depicted in Figure 4. Host cells having a vector chromosomally integrated into an obligatory essential nucleic acid sequence are not viable, whereas integration mutants in facultatively essential or non essential nucleic acid sequences are viable. In order to identify viable clones, the transformed microbial cells are grown under suitable growth conditions and the viable clones are selected for the further procedure.

The method as described can be applied to any suitable microorganism, e.g. bacterium, of interest. Therefore, an preferred embodiment of the present invention relates to a method, wherein said microbial cells are selected from Gram negative and Gram positive bacteria, particularly Enterobacteriaceae, Pseudomonadaceae, Vibrionaceae, Neisseriaceae, Chlamydiaceae, Micrococcaceae, Mycobacteriaceae, Pasteurellaceae, Clostridium or Spirochaetales, and more particularly Salmonella spec., Pseudomonas aeruginosa, Vibrio cholerae, Neisseria gonorrhoeae, Chlamydia trachomatis, Streptococcus pneumoniae, Haemophilus influenzae or Leptospira interrogans.

**Step (b) comprises cultivating the mutant cells and obtaining a library of viable mutant cells.**

By using a genome saturating mutagenesis method as described, two different types of cells having mutations in essential nucleic acid sequences are obtained: Mutants with an insertion in an obligatory essential nucleic acid sequence which are not viable and mutants with an insertion in a non-obligatory essential nucleic acid sequence which are viable. The term "obligatory essential nucleic acid sequence" as used herein means that an intact copy of such a sequence is necessary for microbial survival and growth.

In order to obtain viable mutants for further characterization, the mutagenized cells of step (a) are cultivated. Mutants with an insertion in an obligatory essential nucleic acid sequence are lethal. The remaining mutants are collected and constitute a library of viable mutant microbial cells which can be subjected to the further steps of the claimed method.

**Step (c) comprises contacting permissive eukaryotic host cells with a single clone of the library of step (b) under conditions, wherein an internalization of the microbial cells into the eukaryotic host cells can take place**.

In order to identify mutated microbial cells having a facultatively essential phenotype, permissive eukaryotic host cells are contacted with mutated microbial cells. Permissive eukaryotic cells are cells which can internalize microbial cells, e.g. bacteria. The internalization may be a take up or an infection process or a combination thereof. Take up thereby relates to a process actively mediated by the host cells whereby infection is a process actively mediated by the microbial cells. The permissive eukaryotic host cells are preferably provided in a plurality of separate contacting vessels, e.g. wells of a microtiter plate, reaction tubes etc. and each of the separate contacting vessels is inoculated with a single clone of the mutant library.

For infection, freshly isolated primary cells as well as clonal secondary cells or immortalized cells can be used. Permissive eukaryotic host cells are preferably mammalian cells and particularly human or mouse cells, e.g. selected from macrophages, epithelial cells, fibroblasts, endothelial cells, dendritic cells or muscle cells. For example, human gastric epithelial cells may internalize Enterobacteriaceae, human monocytes may internalize Spirochaetales, human endometrial gland epithelial cells or human cervix carcinoma cells may internalize Chlamydiaceae, pharyngeal carcinoma cells or human umbilical vein endothelial cells may internalize Gram positive cocci, or macrophages may internalize Neisseriaceae.

The amount of microbial cells which are internalized or taken up by the eukaryotic host cells depends on the specific host-microbial system and the ratio of microbial cells to host cells used for infection. Preferably 1 to 100 clonal microbial cells of step (b) are used per permissive eukaryotic host cell in step (c), e.g. 40 to 60 microbial cells per epithelial cell or 1 to 20 microbial cells per macrophage.

In order to permit a sufficient rate of infection the conditions of incubation of mutant microbial cells with eukaryotic host cells should be such that the internalization process is established and/or substantially completed. To differentiate between reduced capacity of infectivity and intracellular survival and/or propagation the contacting time is preferably kept below the intracellular replication time of microbial cells. In accordance with the above, a preferred embodiment of the invention relates to a method, wherein contacting said permissive eukaryotic host cells in step (c) takes between 15 min and 2 hours, particularly about 30 (between 20 and 40 min) min for macrophages or about 1 hour (between 40 min and 80 min) for epithelial cells.

**Step (d) comprises removing non-intracellular microbial cells from the eukaryotic host cells.**

Removing the non-intracellular microbial cells from their eukaryotic host cells after infection is preferably substantially quantitatively performed by washing steps and/or the use of antimicrobial agents.

When using an antimicrobial agent the conditions, e.g. concentration and/or incubation time and/or incubation temperature are preferably selected such that the agent substantially does not enter the host cell. Concentrations of antimicrobial agents are especially important when using phagocytic cells since they take up compounds from the environment by phagocytosis and pinocytosis. Therefore a too high antibiotic concentration can lead to an undesired damage of intracellular microbial cells. When using gentamycin as antibiotic concentrations up to $50\mu g/ml$ are preferred. For example, incubating the eukaryotic host cells with an antimicrobial agent in step (d) is carried out between 5 min and 24 hours, preferably about 15 min (between 5 min and 2 hours) for epithelial cells or about 15 hours (between 30 min and 24 hours) for macrophages.

**Step (e) comprises identifying the microbial cells having reduced capacity of infectivity and/or intracellular survival and/or propagation in permissive eukaryotic host cells.**

To identify a microbial cell which is defect in a facultatively essential nucleic acid sequence, and optionally to determine its level of attenuation or defect in virulence, its features have to be differentiated from those of a respective control cell. Control cells may be wild-type cells and/or known mutant cells, selected from cells of the same or a closely related microorganism having a defined gene mutation so that a certain facultatively essential phenotype is constituted.

After removing mutated microbial cells which did not internalize, the eukaryotic host cells are cultivated under conditions to allow intracellular propagation of the microbial cells. Thereby microbial cells which are not mutated in a facultatively essential gene show wild-type like intracellular survival and/or propagation characteristics, whereas cells which are mutated in a facultatively essential gene exhibit different characteristics, e.g. a reduced growth rate or no growth at all. Thus, a preferred embodiment of the present invention relates to a method, wherein identifying the microbial cells in step (e) comprises incubating the eukaryotic host cells under conditions, wherein propagation of intracellular microbial cells can take place and subsequently determining the presence and/or amount of microbial cells within the eukaryotic host cells.

To recover intracellular microbial cells the eukaryotic host cells may be lysed, e.g. with a detergent which does not destroy the intracellular microbial cells. Preferably lysis is conducted with agents that lyse and/or perforate the eukaryotic membrane but not the microbial cell wall or cytoplasma membrane and/or by sonification and/or freeze-thaw cycles or combinations thereof. Thus, in a preferred embodiment the determining step comprises lysing the eukaryotic host cells of step (d) under conditions substantially permitting the survival of the microbial cells within the eukaryotic host cells and thereby obtaining viable microbial cells.

Microbial cells obtained from the lysate may be further cultivated. Thereby, at least a predetermined portion of the lysate is incubated under conditions allowing proliferation of the microbial cells, e.g. under conditions in which the microbial cells are able to propagate, e.g., incubation in a suitable growth medium under suitable temperature and atmosphere conditions optionally under selective pressure for the mutation. Usually microbial cells having a defect in a facultatively essential gene are recovered in lower amounts from the host cells than control cells such as nonmutated microbial cells or mutants in a facultatively non essential gene or they are not recovered at all. Therefore microbial cells with a defect in a facultatively essential gene are reaching the logarithmic growth phase at a later time point or not at all compared to control cells. Cells having a defined mutation in a facultatively essential gene as well as with a defined mutation in a facultatively non essential gene may be used as control cells, propagated in permissive eukaryotic host cells and compared in their growth behaviour with the mutated cells. Those defined mutations have a distinct and well characterized phenotype in respect to infectivity and/or intracellular survival and/or propagation in permissive eukaryotic host cells. Comparison of the growth of the microbial cells mutants with the growth of the control cells thereby enables a rapid classification of different mutations. Thus, preferably the invention relates to a method further comprising determining the growth of said viable microbial cells and comparing said growth to the growth of control cells.

Measuring the optical density at a time point wherein the control cells with a mutation in a facultatively non essential gene are in the logarithmic growth-phase results in the detection of facultatively essential microbial cells due to their lesser optical density. In accordance with the above, in a further preferred embodiment, the invention relates to a method, wherein the growth is determined by monitoring the optical density of said microbial cells in a non-static growth phase, particularly in an exponential growth phase.

Depending on their growth behaviour compared to corresponding control cells, the mutant microbial cells can be classified with regard to their level of attenuation without using an animal model.

**Step (f) comprises characterizing the obtained nucleic acid sequences and/or polypeptides encoded thereby associated with the reduced capacity of infectivity and/or intracellular survival and/or propagation of the mutant microbial cells in permissive eukaryotic host cells.**

Further characterization of the identified nucleic acid sequences and/or polypeptides encoded thereby which are facultatively essential may be performed via comparative genomics. Comparative genomics may comprise the identification of orthologs.

The term "orthologs" as used herein means nucleic acid sequences that are related by vertical descent from a common ancestor by species diversification and may encode proteins with the same or a similar function in different species. Usually, orthologs retain the same function in the course of evolution. However, orthologous genes may or may not be responsible for a similar function (for review see the glossary of the "Trends Guide to Bioinformatics", Trends Supplement 1998, Elsevier Science).

Orthologous genes, nucleic acids or proteins comprise genes, nucleic acids or proteins which have one or more sequences or structural motifs in common, for example protein binding boxes or structure forming boxes. The sequence motifs of proteins can comprise short, i.e. repetitive sequences or amino acid positions conserved in the primary structure and/or conserved in higher protein structures, e.g., secondary or tertiary structure. Methods like BLAST searches for the identification of a candidate ortholog of a gene or a polypeptide are known to those skilled in the art.

As described, facultatively essential microbial nucleic acid sequences can be used to identify attenuating mutations which can be used as live vaccines. For priorization, comparative genomics can be performed using the results of step (e), wherein the identified and characterized facultatively essential nucleic acids and/or polypeptides sequences are further compared to other microbial amino acid sequences and classified into 3 categories: i) into sequences which are found to a have a defined function and are already known to be suitable for attenuation of the respective microorganism, which are of not much interest, ii) into sequences with a known function but presently unknown suitability for attenuation, which are of medium interest and iii) into sequences with a so far unknown function and unknown suitability for attenuation, which are of high interest. An aspect of the invention therefore relates to the use of the method of the invention for the identification and/or priorization of attenuated microbial cells, particularly bacteria.

The identified and characterized facultatively essential nucleic acids and/or polypeptides sequences may be compared to other microbial amino acid sequences and to sequencs of eukaryotes, also humans, via data base search. Nucleic acid sequences and/or polypeptides encoded thereby which are found in many or all pathogens but not in eukaryotes are preferred targets for broad range antibiotics. Nucleic acid sequences and/or encoded polypeptides for which homologue and/or ortholog sequences are found in no or only a few other pathogens can be selected as targets for narrow-range antibiotics. In accordance, another aspect of the present invention relates to the use of the method according to the invention for the identification and/or priorization of drug targets in microbial cells, particularly bacteria.

A further aspect which is not part of the present invention relates to a nucleic acid sequence, obtainable by the method according to the invention comprising a microbial nucleic acid sequence which is essential for infectivity and/or intracellular survival and/or propagation in eukaryotic host cells. Preferably, the nucleic acid sequence comprises a coding sequence encoding at least the mature form of a polypeptide or protein, i.e. the protein which is post-translationally processed in its biologically active form, for example due to cleavage of leader and/or secretory sequences and/or a prosequence or other natural proteolytic cleavage sites.

Preferably the nucleic acid sequence comprises (a) a sequence derived from an insertion site as shown in Table 1 or Table 2 including a region up to 5 kbp upstream or downstream or preferably 1 kbp upstream or downstream from the insertion site, the complement thereof, an ortholog or a protein coding fragment thereof, (b) a sequence within the scope of the degeneracy of the genetic code of the sequence of (a) or (c) a sequence hybridizing under stringent conditions with the sequence of (a) and/or (b).

Stringent hybridization conditions in the sense of the present invention are defined as those described by (Sambrook, 1989). According to this, hybridization under stringent conditions means that a positive hybridization signal is still observed after washing for 1 hour with 1 x SSC buffer and 0.1 % SDS at 55°C, preferably at 62°C and most preferably at 68°C, in particular, for 1 hour in 0.2 x SSC buffer and 0.1% SDS at 55°C, preferably at 62°C and most preferably at 68°C.

A further aspect which is not part of the present invention therefore relates to a nucleic acid or polypeptide array, comprising at least two, preferably at least 10 of the nucleic acid sequences as described or polypeptides encoded thereby, preferably in an immobilized form. The array preferably comprises a plurality of nucleic acid or polypeptide sequences each in a specific area immobilized on a solid support, e.g. glass, silicon, plastic etc. The array, e.g. a chip may be used for diagnostic purposes or in a screening procedure for identifying new drugs.

In a still further aspect which is not part of the present invention also relates to a vector, comprising at least one of the nucleic acid sequences described or a fragment thereof. Further, a cell transformed with a nucleic acid or the recombinant vector as described is provided. The cell may be a prokaryotic cell such as a Gram negative or Gram positive cell. The cell is not part of the present invention.

A further aspect which is not part of the invention relates to a library of viable mutant cells, comprising a plurality of microorganism clones as obtainable from step (b) of the claimed method. An even further aspect which is not part of the invention relates to an isolated clone of said library, comprising a mutation in a nucleic acid sequence as described.

The method of the present invention provides means for the identification of facultatively essential nucleic acid sequences in microorganisms. Furthermore, it provides means for the identification and characterization of polypeptides or fragments thereof, encoded by said nucleic acids. An other aspect which is not part of the invention therefore relates to a polypeptide, (a) encoded by a nucleic acid sequence as described, a fragment or derivative thereof or (b) encoded by a sequence which is 60%, preferred 65% and particularly preferred 70% homologous to a nucleic acid sequence as described, a fragment or derivative thereof.

Percent (%) homology are determined according to the following equation:

$$H = \frac{n}{L} \times 100$$

wherein H are % homology, L is the length of the basic sequence and n is the number of nucleotide or amino acid differences of a sequence to the given basic sequence.

The terms "fragment" or "derivative" denotes any variant the amino acid deviates in its primary structure, e.g., in sequence composition or in length as well as to analogue components. For example, one or more amino acids of a polypeptide may be replaced in said fragment or derivative as long as the modified polypeptides remain functionally equivalent to their described counterparts.

One or more of the polypeptides as described may be complexed with one or more other polypeptides which may be homologous or heterologous polypeptides *in vivo.* Those proteins are called oligomers, and their single components are called subunits. An example for an heteromeric polypeptide is the *E. coli* membrane-bound enzyme nitrate reductase which contains three subunits. Furthermore, metalloenzymes contain the inorganic cations as stably bound components of the enzyme complex.

Enzymes often are dimers or polymers consisting of homologous and/or heterologous proteins. Thus, only a native protein complex might have an enzymatic activity. To provide such a protein complex, monoclonal or polyclonal antibodies against one of the subunits of the complex can be generated. If antibodies are provided with an affinity tag, the protein complexed with other proteins can be separated from other cellular components by affinity chromatography. Therefore, complexes of polypeptides can be subjected to immunoprecipitation, e.g. with antibodies as described, in order to identify homologous or heterologous polypeptides which might be associated. Accordingly, another aspect which does not form part of the invention relates to a complex of at least one polypeptide or a fragment thereof with at least one other polypeptide.

A further aspect which is not part of the present invention relates to an antibody which is specific for an aforementioned polypeptide or fragment thereof. Antibodies directed against a polypeptide as described may be prepared by any of a variety of methods using immunogens or epitopes of the polypeptide. Such immunogens include the full length polypeptide (which may or may not include the leader sequence) and fragments such as the ligand binding domain, the extracellular domain and the intracellular domain. These antibodies can be monoclonal antibodies, polyclonal antibodies or synthetic antibodies as well as fragments of antibodies, such as Fab+, Fv, F(ab')2, disulphide-bridged Fv or scFv fragments, etc. Monoclonal antibodies can be prepared, for example, by the techniques as originally described by Köhler and Milstein, or in Harlow and Lane "Antibodies, A Laboratory Manual", CSH Press, Cold Spring Harbor, 1988.

The present invention provides means for the identification of a variety of potential live vaccines with different levels of attenuation, which subsequently serve as platforms for the development of recombinant bacterial carriers that can be used to express antigens from heterologous pathogens, thus serving as multivalent vaccines. Thus, a further aspect which is not part of the present invention relates to an attenuated microbial cell comprising at least one of the described nucleic acid sequences, wherein at least one of said nucleic acid sequences is inactivated and wherein said inactivation results in an attenuation/reduction of virulence compared to the wild-type of said cells.

The attenuated microbial cell is selected from a variety of different Gram positive and Gram negative bacteria. Accordingly, a preferred aspect which is not part of the invention relates to an attenuated microbial cell, wherein said cell is a bacterial cell, particularly selected from Enterobacteriaceae, Pseudomonadaceae, Vibrionaceae, Neisseriaceae, Chlamydiaceae, Micrococcaceae, Mycobacteriaceae, Pasteurellaceae, Clostridium or Spirochaetales, and more particularly Salmonella spec., Pseudomonas aeruginosa, Vibrio cholerae, Neisseria gonorrhoeae, Chlamydia trachomatis, Streptococcus pneumoniae, Haemophilus influenzae or Leptospira interrogans.

A nucleic acid sequence or a vector as described above can be used for the identification of an antimicrobial agent. For example the nucleic acid sequence as described can be either directly used as drug target for binding of e.g. an antisense compound, or its encoded polypeptide can be used for binding of natural or synthetic compounds.

The term "compound" as used herein comprises both natural and synthetic molecules, nucleic acids such as antisense molecules, vectors comprising antisense molecules or nucleic acid sequences encoding an antagonist or inhibitor, cells comprising antisense molecules or nucleic acids encoding an antagonist or inhibitor, peptides, polypeptides, proteins, proteinaqueous or non proteinaqueous compounds and/or antibodies. Compounds or plurality of compounds can be identified from large libraries of both natural product and synthetic (or semi-synthetic) extracts or chemical libraries according to methods known in the art.

Furthermore the vector can be used for expression of the respective nucleic acid sequence and subsequently binding assays. In an other approach the nucleic acid sequence as described can be used for the prepartation of an attenuated microbial cell which can either serve as monovalent live vaccine or as bi- or mulitvalent carrier of heterologous antigens. Means for the preparation of an attenuated microbial cell belong to state of the art and are selected from deleting, mutating, fusing, inserting and other a sequence which is known to be attenuating. Accordingly, the vector as described can be used. A further aspect which is not part of the invention therefore relates to the use of a nucleic acid sequence or a vector, comprising at least 50 nucleotides of a nucleic acid sequence as described for the identification of an antimicrobial agent and/or for the preparation of an attenuated cell, a live vaccine or a carrier for the presentation of an antigen to a host. A preferred aspect relates to the use of a nucleic acid sequence or a vector as described, wherein at least one coding region of said sequence is functionally deleted.

A nucleic acid sequence as described can be directly used as a target for an antimicobial drug. A specific drug may bind to the facultatively essential nucleic acid and thereby functionally inactivate the nucleic acid, so that the affected microorganism is affected in its infectivity, survival and/or propagation in a host cell and finally gets eliminated. Accordingly, a further aspect which is not part of the invention relates to the use of a nucleic acid sequence as described for the identification of an antimicrobial compound.

In order to identify drugs which are suitable as broad range antibiotics or rather to the contrary as narrow range antibiotics, the nucleic acid sequences can be used for the identification of corresponding targets in other microorganisms. This can be performed as described by comparative genomics. In accordance, still an other aspect which is not part of the present invention relates to the use of a nucleic acid for the identification of a homologous nucleic acid in other microorganisms.

As described above, attenuated microbial cells can be identified and/or priorized by the method of the present invention. Thereby, either the library of viable mutant microbial cells as described or a clone thereof or a nucleic acid sequence which can be obtained from that library or clone can be used. The library of viable mutants can be regarded as library of different attenuated microbial cells, whereas the nucleic acid sequence which is obtainable therefrom can be used to identify the function of the regarding gene and its potential presence in other microorganisms. An aspect which is not part of the present invention therefore relates to the use of the library of as described or a clone or a nucleic acid sequence obtainable from said library or said clone for the identification and/or priorization of attenuated microbial cells, particularly bacteria and/or the identification and/or priorization of drug targets in microbial cells, particularly bacteria.

A facultatively essential nucleic acid sequence as described and a polypeptide encoded thereby can be used as a drug target. An aspect which is not part of the present invention therefore also relates to the use of a polypeptide or a complex thereof as a drug target. In accordance, a complex of at least one polypeptide with at least one other polypeptide can be an adequate target for drugs.

In another aspect which is not part of the present invention relates to the use of an antibody for the identification of a drug target, wherein said drug target comprises at least one polypeptide or a fragment thereof as described, optionally complexed with at least one other polypeptide. An antibody as described above can be used for immunoprecipitations in order to identify polypeptide complexes which are suitable as drug targets.

As described, a cell which is attenuated in a nucleic acid sequence which is obtained by the method of the present invention can be used for the preparation of a a live carrier for the expression of at least one heterologous antigen or of a live vaccine. Accordingly, an other aspect which is not part of the invention relates to the use of an attenuated cell as described for the preparation of a live carrier for the expression of at least one heterologous antigen or of a live vaccine. An even further aspect which is not part of the invention is a method for the preparation of a live vaccine, comprising providing a living microbial cell comprising a nucleic acid sequence as described and inactivating at least one of said sequences to obtain an attenuated microbial cell as described.

In the present invention a novel method for the identification of facultatively essential nucleic acid sequences, in particular microbial sequences, and polypeptides encoded thereby is provided. The nucleic acids, polypeptides encoded thereby and/or corresponding complexes can be used as drug targets and/or for the identification of drugs. Another aspect which is not part of the invention therefore relates to a method for the screening and/or identification of an antimicrobial compound, wherein a nucleic acid sequence as described and/or polypeptide or corresponding fragment or derivative thereof and/or a complex as described is used.

An antimicrobial compound as described or an attenuated microbial cell can be used for the preparation of a pharmaceutical composition. Accordingly, a still further aspect which is not part of the present invention is a pharmaceutical composition, comprising as an active agent (a) a nucleic acid sequence as described, or (b) a vector, (c) a cell, (d) an antibody and/or (e) an attenuated cell as described, particularly as an immunologically protective live vaccine, optionally in a physiologically acceptable diluent or carrier. Physiologically acceptable diluents or carriers are known from state of the art.

The pharmaceutical composition as described can contain further active components. An aspect, therefore relates to a pharmaceutical composition, comprising a further ingredient, e.g. an antibiotic and/or cytokine.

As described, the identification of a facultatively essential microbial sequence can be used for the preparation of a pharmaceutical composition which can be used as antiinfectivum or antibiotic. Therefore, an other aspect which is not part of the present invention relates to the use of a pharmaceutical composition as described for the prevention and/or therapy of microbial infections.

The following examples serve to explain the method of the invention in more detail.

## Examples

### Cell culture:

J774A.1 macrophages are cultivated in Dulbecco's modified Eagle's medium (DMEM) supplemented with 10% heat-inactivated fetal calf serum (FCS) at 37° C in 10% $CO_2$. Caco-2 epithelial cells are grown in minimal essential medium (MEM) supplemented with essential amino acids and 20% heat-inactivated FCS, HeLa epthelial cells are grown in RPMI medium supplemented with 5% heat-inactivated FCS at 37° C in 5% $CO_2$. Cells are seeded into 96-well microtiter plates at a concentration of 5 x $10^4$ cells per well the day before. J774A.1 mouse monocytes-macrophages are supplied by DSMZ (Deutsche Sammlung von Mikroorganismen und Zellkulturen) No. ACC 170, Caco-2 human colon adenocarcinoma cells by DSMZ No. ACC 169 and Hela human cervic carcinoma cells by DSMZ No. ACC 57.

### Bacteria:

Construction of a genome saturating mutant bank of *S. typhimurium* is described elsewhere (WO 02/83940). Briefly, genome-representing fragments of 350-500 base pairs are generated from chromosomal DNA of *Salmonella enterica* serovar Typhimurium using random primers. Fragments are cloned into the Kpnl-restricted conditionally replicating vector pIDM001 (Fig. 3), transformed into *repA*-wild-type E. coli strain EC101 and grown under permissive temperature (30°C) in the presence of 17.5 μg/ml tetracycline. Plasmid-DNA is isolated and transformed into *S. enterica* serovar Typhimurium. Insertional duplication mutagenesis by homologous recombination occurs during bacterial growth at permissive temperature in LB-medium (tetracyclin 17.5 μg/ml). After a shift to non-permissive temperature. (38.5°C) mutants having two different phenotypes are obtained: mutants having a viable or a non viable phenotype. *Salmonella* mutants having an obligatory essential phenotype are non-viable, whereas mutants having an facultatively essential or non essential phenotype are viable.

Colonies with a viable genotype are grown overnight in 150 μl of LB broth (17.5 μg/ml tetracycline) in microtiter dishes at 37°C with shaking. Overnight cultures are diluted and grown another 6 hours to late logarithmic phase for infection of macrophages. For infection of epithelial cells colonies are grown in 300 μl LB broth under microareophilic conditions. Optical density (OD) is measured and bacterial dilutions are calculated to infect the macrophages with a multiplicity of 10 bacteria per macrophage and 50 bacteria per epithelial cell.

**Infection Assay:**

The eukaryotic host cells are previously washed with phosphate buffered saline and infection is performed in doublets, wherein 100 μl of the bacterial suspension in medium are added to the cells. In order to synchronize the infection process, host cells and bacteria are centrifuged within the microtiter plate for 5 min at 500 rpm. The amount of mutant bacterial cells of a single clone derived from the mutation assay per host cell is thereby calculated as 10 bacteria per macrophage and 50 bacteria per epithelial cell.

After centrifugation, the plates are incubated for 30 min (macrophages) or 1 h (epithelial cells).

Removing non intcracellular bacterial cells is performed by washing steps with PBS and the additional adding of gentamicin. Gentamicin is used at a final concentration of 10 μg/ml in 100 μl DMEM growth medium for an incubation time of 15 h for macrophages and at a concentration of 100 μg/ml for 15 min for epithelial cells. After the treatment with gentamicin, cells are washed with PBS and lysed with 0.5% sodium deoxycholate. An aliquot of the lysis mix, containing the microbial cells is diluted in LB broth (17.5 μg/ml Tetracyline) in a microtiter dish and the bacteria grown at 37 °C until logarithmic phase of the wild-type control is reachend. Optical density (OD) is measured and every microbial cell showing a decrease in optical density compared to wild-type Salmonella can be selected as a possible attenuating mutant. In particular every microbial cell with the same or a lesser OD than a known Salmonella mutant (*aroA* for macrophages, *prgH* for epithelial cells) is selected for subsequent analysis.

**Characterizing facultatively essential nucleic acid sequences:**

Characterizing obligatory essential nucleic acid sequences is described in WO 02/83940. The characterization of facultatively essential nucleic acid sequences is performed accordingly with the following modifications: Colonies with a facultatively essential genotype are characterized further by PCR using two plasmid specific primers which bind at both sides of the cloning site of the vector and the PCR product is sequenced. The obtained sequence is analyzed for vector-specific and fragment-specific nucleotides. Only fragment specific nucleotides are devised to data analysis using the following data bases: http://genome.wustl.edu/gsc/

http://www.ncbi.nlm.nih.gov/

http://www.sanger.ac.uk/

http://www.genome.wisc.edu/

BLAST searches using the above mentioned data bases are performed to identify homologous nucleotide sequences. All sequences are compared to the *Salmonella enterica* serovar Typhimurium, *S. typhi, Neisseria, Shigella, Yersinia, Staphylococcus, Streptococcus, Chlamydia, Vibrio* and *E. coli* genome sequence, respectively. Table 1 defines the part of a *Salmonella enterica* serovar *Typhimurium* genomic sequence to which the sequenced fragment is identical or nearly identical (insertion region). Thus, the gene complex and gene organization of a *Salmonella enterica* serovar *Typhimurium* subgenomic fragment where an insertion into a facultatively essential gene has taken place is identified.

Next, the targeted open reading frame (ORF) is identified in accordance with the annotations of the *Salmonella enterica* serovar Typhimurium , *E. coli* or *S. typhi* genomes. In addition, the translated sequence of any putative ORF deduced from the sequenced fragment including its flanking regions is compared to the data bases. Identified homologies and operon structures deduced from annotated sequences and/or from experimental studies are stored. Other insertional mutants within the same operon are taken into consideration to identify the obligate essential gene within a gene complex (Table 1: Facultatively essential genes).

To identify homologous proteins or domains, the amino acid sequences of the identified facultatively essential nucleic acid sequences are compared to other microbial or eukaryotic genomes using the above mentioned databases (Table 1: Homologies).

The insertion sites listed in Table 1 are showing the positions in the genome of Salmonella enterica ssp. Typhimurium (BLASTN (DNA query sequence) against S. typhimurium database; http://genome.wustl. edu/gsc/Blast/client.pl.

The following list depicts the record date.

Parent Directory:

19-April-2001: 11,08; 11,17; 21,01; 21,08; 11,89; 12,04; 12,48; 12,62;

20-April-2001: 11,06; 11,07; 12,10;

27-April-2001: 12,20; 12,55; 12,57; 12,68; 12,70; 12,80; 12,78; 12,85; 13,24; 13, 34; 13, 84; 16,24;

03-May-2001: 21,90; 24,96; 26,21; 26,32; 26,83; 26,55; 26,60; 30,13; 30,77;

09-May-2001: 36,05; 36,51; 40,12; 40,93;49,60; 49,64; 49,80;

15-May-2001: 40,86.

Table 2 describes the insertions into the genome of Salmonella enterica serovar Typhimurium by homologous recombination via a cloned fragment. The first lane contains the clone number. The second lane contains the information whether the respective mutant was attenuated in a macrophage survival assay (M) or in an infectious assay with epithelial cells (E) or both (M/E). The third and fourth lane describe the start and the end of the sequenced region of the cloned insertion fragment with respect to the Salmonella enterica serovar Typhimurium genome sequence. The fifth lane defines the start of the sequence, e. g. "a" indicates that the sequence of the cloned fragment starts with the nucleotide given in the lane "insertion site a" . (p) in front of a nucleotide sequence position refers to the sequence of the plasmid pSLT.

The clones described in Tables 1 and 2 are not part of the invention.

**Literature**

**Claus,** H., M. Frosch, et al. (1998). "Identification of a hotspot for transformation of Neisseria meningitidis by shuttle mutagenesis using signature-tagged transposons." Mol Gen Genet 259(4): 363-71.

**Drevets,** D. A., B. P. Canono, et al. (1994). "Gentamicin kills intracellular Listeria monocytogenes." Infect. Immun. 62(6): 2222-8.

**Fields,** P. I., R. V. Swanson, et al. (1986). "Mutants of *Salmonella typhimurium* that cannot survive within the macrophage are avirulent." Proc Natl Acad Sci U S A 83(14): 5189-5193.

**Hensel,** M., J. E. Shea, et al. (1995). "Simultaneous identification of bacterial virulence genes by negative selection." Science 269(5222): 400-3.

**Köhler,** G., and C. Milstein. (1975). "Continuous cultures of fused cells secreting antibody of predefined specificity". Nature. 256:495-7.

Mahan, M. J., J. M. Slauch, et al. (1993). "Selection of bacterial virulence genes that are specifically induced in host tissues." Science 259(5095): 686-8.

**Mahan,** M. J., J. W. Tobias, et al. (1995). "Antibiotic-based selection for bacterial genes that are specifically induced during infection of a host." Proc Natl Acad Sci U S A 92(3): 669-73.

**Polissi,** A., A. Pontiggia, et al. (1998). "Large-scale identification of virulence genes from *Streptococcus pneumoniae."* Infect Immun 66(12): 5620-5629.

**Sambrook,** J., E. F. Fritsch, and T. Maniatis. (1989). Molecular cloning: a laboratory manual, 2nd ed. Cold Spring Harbor Laboratory, Cold Spring Harbor, N. Y.

**Schena,** M., D. Shalon, et al. (1995). "Quantitative monitoring of gene expression patterns with a complementary DNA microarray." Science 270(5235): 467-70.

**Valdivia,** R. H. and S. Falkow (1996). "Bacterial genetics by flow cytometry: rapid isolation of *Salmonella typhimurium* acid-inducible promoters by differential fluorescence induction." Mol Microbiol 22(2): 367-378.

**Valdivia,** R. H. and S. Falkow (1997). "Fluorescence-based isolation of bacterial genes expressed within host cells." Science 277(5334): 2007-11.

Table 1

| Clone No. | Attenuated in Epithelial cells | Attenuated in Macrophages | Insertion region | Facultatively essential gene | Function | Homologies |
|---|---|---|---|---|---|---|
| 12,35 | | yes | 184437-184568 | yacH | putative membrane protein | highly conserved among bacteria |
| 12,48; 12,04; 12,62; 86,56; 83,57 | yes | yes | 433884-433966 | ddlA | D-Alanin:D-Alanin Ligase | highly conserved among bacteria |
| 12,65; 12,85 | | yes | 3227511-3227639 | yggE | putative actin | conserved in Enterobacteria ceae |
| 15,75 | | yes | 815498-815625 | unknown ORF between tolB and tolR | | highly conserved among Salmonella |
| 17,17 | | yes | 607824-607954 | fimD | Fimbria | highly conserved among bacteria |
| 17,62 | | yes | 1512469-1512600 | GloA | S-D-lactoylglutathione methylglyoxal lyase | highly conserved among bacteria |
| 17,84; 18,08 | | yes | 1474094-1474232 | ORF 319 | SPI-2 | highly conserved among Salmonella |
| 18,02 | | yes | a) 2733166-2733317 b) 1963305-1963456 | a) unknown ORF b) mig-3 | b) possible DNA inversion system; macrophage-inducible | highly conserved among Salmonella |
| 18,60 | | yes | 1197381-1197513 | rssC | Overexpression of RssC prevents RpoS turnover in Salmonella typhimurium | highly conserved among Salmonella |
| 19,71; 46,24; 46,59 | | yes | 4271408-4271511 | yiiP | putative transport; Not classified | highly conserved among bacteria |

| Clone No. | Attenuated in Epithelial cells | Attenuated in Macrophages | Insertion region | Facultatively essential gene | Function | Homologies |
|---|---|---|---|---|---|---|
| 20,77 | | yes | 2024618-2024751 | l)otsB 2)trehalose biosynthesis operon | 1)trehalose-6-phosphate phosphatas | highly conserved among bacteria |
| 20,93; 33,59 | | yes | 2830134-2830270 | | proline/threonine-rich protein | highly conserved among bacteria |
| 23,30 | | yes | 2567763-2567880 | eutB | ethanolamine ammonia-lyase | highly conserved among bacteria |
| 23,69; 24,60 | | yes | 1569827-1569948 | unknown ORF | | highly conserved among Salmonella |

| | | | | | | |
|---|---|---|---|---|---|---|
| 23,70 | | yes | 1872978-1873114 | hemK | possible protoporphyrinogen oxidase;Biosynthesis of cofactors, carriers: Heme, porphyrin | highly conserved among bacteria |
| 24,40 | | yes | 1401921-1402050 | b1728 | unbekannt | highly conserved among bacteria |
| 25,10 | | yes | 514740-514842 | cof | keine Angabe | highly conserved among bacteria |
| 26,21; 26,32; 26,83 | | yes | 2098911-2099039 | cobS | cobalamin synthase | highly conserved among bacteria |
| 30,91 | | yes | 1951073-1951198 | unknown ORF | | highly conserved among Salmonella |
| 31,31 | | yes | 2318643-2318690 | yejB | | highly conserved among bacteria |
| 31,67 | | yes | 1768724-1768848 | unknown ORF | | highly conserved among Salmonella |
| 31,84 | | yes | 2162750-2162900 | manB | phosphomannomutase | highly conserved among bacteria |
| 34,10; 46,06; 46,34 | | yes | 1950807-1950948 | pphA | protein phosphatase 1 modulates phosphoproteins, signals protein misfolding | highly conserved among bacteria |
| 34,59; 34,62 | | yes | 1655654-1655790 | 1) aac(6')-ly 2) nmpC | 1)minoglycoside 6'-N-acetyltransferase ) outer membrane porin protein | highly conserved among bacteria |
| 34,85 | | yes | 1694084-1694230 | pathogenicity islet in enteritidis | So667; putative collagenase; similar to Escherichia coli hypotheitcal protein o667 | highly conserved among Salmonella |
| 38,32 | | yes | 4475230-4475388 | uvrA | DNA repair enzyme | highly conserved among bacteria |
| 38,53; 43,20 | | yes | 432860-433000 | b0379 | orf; Unknown function | highly conserved among bacteria |

| | | | | | | |
|---|---|---|---|---|---|---|
| 38,85; 40,93; 40,20 | | yes | 1550058-1550202 | ompS2 | | highly conserved among bacteria |
| 38,94 | | yes | 1793201-1793367 | yicW | | highly conserved among bacteria |
| 39,16; 40,60 | | yes | 1758446-1758585 | acnE | pyruvate dehydrogenase, decarboxylase component | highly conserved among bacteria |
| 39,23; 39,91 | | yes | 1789033-1789155 | sapF | antimicrobial peptides-resistance locus | highly conserved among bacteria |
| 39,64 | | yes | 1672758-yes 1672877 | unknown ORF | | highly conserved among Salmonella |
| 41,41 | | yes | 4077375-4077449 | atpA | The ATP synthase subunit alpha | highly conserved among bacteria |
| 42,32; 49,64 | | yes | 1281143-1281285 | fabF | enzyme; Fatty acid and phosphatidic acid biosynthesis | highly conserved among bacteria |
| 42,36 | | yes | 1337052-1337193 | unknown ORF | | highly conserved among Salmonella |
| 43,24 | | yes | 1873346-1873491 | prfA | release factor 1 | highly conserved among bacteria |
| 44,47 | | yes | 857476-857635 | hutH | histidine ammonia-lyase (hutH) precursor | highly conserved among bacteria |
| 44,53 | | yes | 2725832-2725968 | lep | Signal peptidas | highly conserved among bacteria |
| 45,41 | | yes | 2701413-2701570 | cadA | lysine decarboxylase | highly conserved among bacteria |
| 46,67 | | yes | 1896451-1896574 | treA | enzyme; Osmotic adaptation | highly conserved among bacteria |
| 53,41 | | yes | 632639-632791 | unknown ORF | | highly conserved among Salmonella |
| 53,83 | | yes | 3036510-3036654 | 1+2)spaM | 2)surface presentation of antigens | highly conserved among bacteria |

(continued)

| | | | | | | |
|---|---|---|---|---|---|---|
| 53,84 | | yes | 3508826-3508978 | nanA | enzyme; Surface polysaccharides and antigens N-acetylneuraminate lyase (aldolase); catabolism of sialic acid | highly conserved among bacteria |
| 55,46 | | yes | 1852436-1852568 | tyrT | tyrosine t-RNA | highly conserved among bacteria |
| 55,70 | | yes | 2191887-2192025 | fcl | GDP-L-fucose synthetase | highly conserved among bacteria |
| 55,71 | | yes | 2545991-2546152 | ptsI | Bacterial Phosphotransferase System | highly conserved among bacteria |
| 57,24 | | yes | 810228-810367 | cydA | cytochrome d oxidase subunit I | highly conserved among bacteria |
| 63,11; 70,60 | | yes | 2017282-2017452 | cheA | sensory protein | highly conserved among bacteria |
| 70,28 | | yes | 1710271-1710428 | ydcG | putative glycoprotein | highly conserved among bacteria |
| 70,62 | | yes | 2280576-2280735 | sfiX | vancomycin resistance at 43C | highly conserved among bacteria |
| 70,64 | | yes | 1128861-1129026 | | lambda phage H tail component homolog | |
| 71,34 | | yes | 1322353-1322488 | ycfB | orf; Unknown function | highly conserved among bacteria |
| 71,36; 71,90 | | yes | 2652772-2652933 | b2511 | putative GTP-binding factor | highly conserved among bacteria |
| 72,76 | | yes | 4271664-4271800 | ORF_o300 | keine Angabe | highly conserved among Salmonella |
| 72,78 | | yes | 2815439-2815583 | trmD | tRNA(mlG37)m ethyltransferase | highly conserved among bacteria |
| 73,29; 73,30 | | yes | 1731811-1731934 | cybB oder ydcF | | highly conserved among bacteria |

(continued)

| | | | | | | |
|---|---|---|---|---|---|---|
| 73,41 | | yes | 641887-642008 | entD | enterobactin biosythesis | highly conserved among bacteria |
| 73,60 | | yes | 1770347-1770466 | unknown ORF | | highly conserved among Salmonella |
| 83,64 | | yes | 3449585-3449695 | pnp | polynucleotide phosphorylase; cytidylate kinase activity | highly conserved among bacteria |
| 83,91 | | yes | 4754463-4754580 | ORF_f248 | | highly conserved among Salmonella |
| 84,10 | | yes | 1514237-1514359 | b1649 | | highly conserved among bacteria |
| 84,25 | | yes | 110970-111108 | hepA | probable ATP-dependent RNA helicase | highly conserved among bacteria |
| 84,68 | | yes | 1340649-1340786 | oxd-6a | | highly conserved among bacteria |
| 85,21 | | yes | 1694100-1694230 | So667 | putative collagenase | highly conserved among bacteria |
| 86,22 | | yes | 1675097-1675231 | b1451 | putative outer membrane receptor for iron transport | highly conserved among bacteria |
| 86,28 | | yes | 3204571-3204684 | xerD | site-specific recombinase | highly conserved among bacteria |
| 86,41 | | yes | 1374996-1375123 | unknown ORF between sspA and selD | | highly conserved among Salmonella |
| 86,57 | | yes | 1499119-1499239 | ssaR | SPI-2 | highly conserved among Salmonella |
| 86,67 | | yes | 1497086-1497203 | ssaO | secretion system apparatus, SPI-2 | highly conserved among bacteria |
| 86,74 | | yes | 104640-104775 | surA | survival protein | highly conserved among bacteria |

(continued)

| | | | | | | |
|---|---|---|---|---|---|---|
| 86,75 | | yes | 1363103-1363235 | yeaG | | highly conserved among bacteria |
| 87,12 | | yes | 1686933-1687050 | b1444 | putative aldehyde dehydrogenase | highly conserved among bacteria |
| 87,16 | | yes | 4440717-4440850 | pgi | phosphoglucose isomerase | highly conserved among bacteria |
| 87,20 | | yes | 4659206-4659330 | unknown ORF | | highly conserved among Salmonella |

table 2

| clone number | assay | insertion site a | insertion site b | sequence start | clone number | assay | insertion site a | insertion site b | sequence start |
|---|---|---|---|---|---|---|---|---|---|
| 0 | M | 0056218 | 0056336 | a | 145,42 | M | 1085405 | 1085517 | b |
| 124,51 | M | 0098846 | 0098966 | a | 111,78 | M | 1118528 | 1118638 | b |
| 86,74 | M | 0104640 | 0104775 | a | 33,21 | M | 1120409 | 1120506 | a |
| 84,25 | M | 0110970 | 0111108 | a | 132,26 | M | 1128556 | 1128666 | a |
| 18,46 | M | 0127020 | 0127165 | b | 70,64 | M | 1128861 | 1129026 | b |
| 49,87 | M | 0178148 | 0178262 | a | 139,09 | M/E | 1155066 | 1155200 | a |
| 12,68 | E | 0180871 | 0181022 | a | 57,65 | M | 1191491 | 1191604 | a |
| 12,35 | E | 0184437 | 0184568 | b | 18,6 | M | 1197381 | 1197513 | b |
| 73,27 | M | 0187768 | 0187888 | a | 83,8 | M | 1218611 | 1218715 | b |
| 51,05 | M | 0197612 | 0197734 | a | 129,05 | M | 1232626 | 1232736 | a |
| 129,6 | M | 0261984 | 0262108 | b | 130,61 | M | 1260868 | 1260991 | b |
| 88,05 | M | 0289248 | 0289396 | a | 57,35 | M | 1263116 | 1263244 | b |
| 119,88 | M | 0292885 | 0293016 | a | 115,64 | M | 1265964 | 1260074 | b |
| 123,57 | M | 0297118 | 0297250 | b | 49,64 | M | 1281143 | 1281292 | a |
| 49,6 | M | 0312767 | 0312910 | b | 90,74 | M | 1292679 | 1292863 | b |
| 12,66 | M | 0336040 | 0336169 | a | 136,86 | M | 1298706 | 1298812 | b |
| 91,29 | M | 0345442 | 0345556 | a | 87,31 | M | 1305333 | 1305431 | a |
| 134,71 | M | 0348388 | 0348498 | a | 135,77 | M | 1316006 | 1316116 | b |
| 38,53 | M | 0432860 | 0433000 | a | 119,83 | M | 1320023 | 1320166 | b |
| 88,67 | M | 0433884 | 0433997 | a | 71,34 | M | 1322353 | 1322488 | a |
| 112,52 | M | 0447051 | 0447161 | a | 67,88 | M | 1323755 | 1323863 | a |
| 129,23 | M | 0455027 | 0455155 | a | 88,38 | M | 1324907 | 1325069 | b |
| 36,51 | M | 0471865 | 0472016 | b | 42,36 | M | 1337052 | 1337193 | a |
| 103,89 | M | 0495452 | 0495550 | a | 58,59 | M | 1339262 | 1339391 | a |
| 89,7 | M | 0497927 | 0498093 | b | 84,68 | M | 1340649 | 1340786 | a |
| 25,63 | M | 0506434 | 0506538 | b | 25,86 | M | 1341766 | 1341901 | a |
| 25,1 | M | 0514740 | 0514842 | a | 26,6 | M | 1351857 | 1351995 | a |
| 101,71 | M | 0537605 | 0537707 | b | 86,75 | M | 1363103 | 1363235 | b |
| 87,17 | M | 0603389 | 0603496 | a | 89,81 | M | 1374302 | 1374473 | a |
| 17,17 | M | 0607824 | 0607954 | b | 86,41 | M | 1374996 | 1375123 | a |
| 11,08 | E | 0624820 | 0624914 | b | 136,27 | M | 1391845 | 1391955 | b |
| 53,41 | M | 0632639 | 0632791 | a | 23,17 | M | 1401913 | 1402047 | a |
| 12,55 | E | 0637368 | 0637443 | a | 134,12 | M | 1406475 | 1406585 | b |
| 73,41 | M | 0641887 | 0642008 | a | 26,55 | M | 1407694 | 1407828 | a |
| 49,56 | M | 0675340 | 0675462 | a | 91,38 | M | 1411678 | 1411788 | a |
| 13,24 | E | 0699565 | 0699654 | b | 58,45 | M | 1419008 | 1419114 | b |
| 46,71 | M | 0706860 | 0706996 | a | 132,82 | M | 1430121 | 1430231 | b |
| 144,51 | M | 0741911 | 0742027 | a | 28,82 | M | 1447598 | 1447733 | a |
| 11,89 | M | 0746889 | 0746985 | a | 28,67 | M | 1457125 | 1457259 | b |

| clone number | assay | insertion site a | insertion site b | sequence start | clone number | assay | insertion site a | insertion site b | sequence start |
|---|---|---|---|---|---|---|---|---|---|
| 62,36 | M | 0781680 | 0781748 | b | 17,84 | M | 1474094 | 1474232 | a |
| 30,96 | M | 0803257 | 0803391 | b | 12,1 | E | 1479487 | 1479574 | a |
| 57,24 | M | 0810228 | 0810367 | a | 28,12 | M | 1481198 | 1481319 | b |
| 15,75 | M | 0815498 | 0815625 | b | 126,77 | M | 1487122 | 1487278 | b |
| 44,47 | M | 0857476 | 0857635 | a | 104,66 | M | 1495296 | 1495364 | a |
| 87,22 | M | 0903393 | 0903469 | b | 86,67 | M | 1497086 | 1497203 | b |
| 83,46 | M | 0951813 | 0951929 | b | 73,17 | M | 1499119 | 1499239 | a |
| 122,24 | M | 1022033 | 1022162 | b | 88,49 | M | 1502694 | 1502813 | a |
| 40,86 | M | 1022234 | 1022380 | a | 17,62 | M | 1512469 | 1512600 | a |
| 40,16 | M | 1027456 | 1027610 | a | 84,1 | M | 1514237 | 1514359 | a |
| 40,68 | M | 1027772 | 1027918 | b | 46,51 | M | 1535563 | 1535711 | a |
| 140,02 | M | 1060566 | 1060694 | a | 61,26 | M | 1549727 | 1549865 | a |
| 23,69 | M | 1569827 | 1569948 | a | 80,67 | M | 1893997 | 1894108 | b |
| 98,03 | M | 1571057 | 1571214 | b | 46,67 | M | 1896451 | 1896574 | b |
| 58,61 | M | 1576630 | 1576747 | a | 68,86 | M | 1900178 | 1900306 | a |
| 116,86 | M | 1582276 | 1582386 | a | 90,94 | M | 1909985 | 1910103 | a |
| 75,72 | M | 1584679 | 1584801 | a | 104,77 | M | 1912905 | 1913035 | a |
| 123,79 | M | 1587573 | 1587700 | b | 105,11 | M | 1914761 | 1914882 | b |
| 136,76 | M | 1595810 | 1595927 | b | 87,49 | M | 1921216 | 1921348 | b |
| 120,1 | M | 1604457 | 1604606 | a | 34,1 | M | 1950807 | 1950948 | b |
| 77,21 | M | 1610374 | 1610484 | a | 134,53 | M | 1980256 | 1980366 | a |
| 124,26 | M | 1618080 | 1618194 | b | 16,24 | E | 1989970 | 1990109 | a |
| 99,5 | M | 1618937 | 1619047 | a | 70,6 | M | 2017282 | 2017451 | a |
| 24,17 | M | 1620090 | 1620242 | a | 20,77 | M | 2024618 | 2024751 | b |
| 101,92 | M | 1632388 | 1632500 | b | 89,08 | M | 2030724 | 2030885 | b |
| 97,81 | M | 1633849 | 1634005 | b | 58,75 | M | 2033161 | 2033275 | b |
| 125,14 | M | 1639965 | 1640082 | b | 89,45 | M | 2098498 | 2098603 | a |
| 34,62 | M | 1655658 | 1655790 | b | 26,83 | M | 2098900 | 2099039 | a |
| 31,55 | M | 1660155 | 1660273 | b | 90,34 | M | 2123344 | 2123480 | a |
| 20,33 | M | 1668821 | 1668947 | b | 127,95 | M | 2138534 | 2138651 | a |
| 39,64 | M | 1672758 | 1672877 | a | 31,84 | M | 2162750 | 2162900 | a |
| 86,22 | M | 1675097 | 1675231 | a | 51,24 | M | 2184921 | 2185063 | a |
| 131,41 | M | 1676539 | 1676649 | a | 55,7 | M | 2191887 | 2192025 | a |
| 41,71 | M | 1680907 | 1681067 | b | 90,27 | M | 2248848 | 2249003 | a |
| 52,84 | M | 1684342 | 1684497 | a | 79,3 | M | 2254275 | 2254379 | .b |
| 87,12 | M | 1686933 | 1687050 | a | 133,94 | M | 2273178 | 2273288 | b |
| 83,25 | M | 1693013 | 1693123 | b | 70,62 | M | 2280576 | 2280735 | b |
| 75,07 | M | 1694067 | 1694230 | b | 144,83 | M | 2285983 | 2286107 | b |
| 102,64 | M | 1695966 | 1696075 | a | 31,31 | M | 2318643 | 2318690 | a |
| 77,25 | M | 1703634 | 1703794 | a | 88,21 | M | 2322527 | 2322645 | b |

| clone number | assay | insertion site a | insertion site b | sequence start | clone number | assay | insertion site a | insertion site b | sequence start |
|---|---|---|---|---|---|---|---|---|---|
| 125,55 | M | 1705801 | 1705939 | b | 12,78 | E | 2337121 | 2337255 | b |
| 70,28 | M | 1710271 | 1710428 | b | 103,74 | M | 2341206 | 2341321 | b |
| 88,42 | M | 1715611 | 1715738 | b | 127,29 | M | 2417297 | 2417407 | b |
| 105,06 | M | 1718745 | 1718875 | b | 137,94 | M | 2428101 | 2428253 | a |
| 95,95 | M | 1731155 | 1731300 | b | 73,26 | M | 2448537 | 2448665 | b |
| 73,29 | M | 1731811 | 1731934 | a | 39,42 | M | 2508052 | 2508170 | a |
| 120,72 | M | 1740205 | 1740205 | a | 124,89 | M | 2510922 | 2511041 | a |
| 103,9 | M | 1754410 | 1754532 | b | 55,84 | M | 2545988 | 2546152 | b |
| 40,6 | M | 1758434 | 1758585 | b | 23,3 | M | 2567763 | 2567880 | a |
| 132,87 | M | 1766705 | 1766815 | a | 145,45 | M | 2589517 | 2589627 | a |
| 11637 | M | 1768367 | 1768477 | b | 87,53 | M | 2601682 | 2601832 | b |
| 31,67 | M | 1768724 | 1768848 | b | 78,62 | M | 2602170 | 2602274 | a |
| 73,6 | M | 1770347 | 1770466 | b | 137,05 | M | 2623492 | 2623629 | b |
| 139,7 | M | 1781035 | 1781173 | a | 51,54 | M | 2647751 | 2647840 | b |
| 39,91 | M | 1789031 | 1789155 | b | 71,36 | M | 2652772 | 2652933 | b |
| 38,94 | M | 1793201 | 1793367 | b | 80,18 | M | 2655404 | 2655493 | b |
| 124,9 | M | 1796732 | 1796871 | b | 67,93 | M | 2698553 | 2698667 | b |
| 119,55 | M | 1824031 | 1824149 | b | 74,43 | M | 2701413 | 2701581 | a |
| 60,49 | M | 1849929 | 1850042 | b | 44,53 | M | 2725832 | 2725968 | a |
| 17,47 | M | 1850020 | 1850171 | a | 55,72 | M | 2728649 | 2728797 | b |
| 38,74 | M | 1857213 | 1857359 | a | 18,02 | M | 2733166 | 2733317 | a |
| 23,7 | M | 1872978 | 1873114 | a | 134,69 | M | 2738611 | 2738721 | a |
| 12,7 | E | 2746299 | 2746432 | a | 99,25 | M | 4377503 | 4377633 | a |
| 52,59 | M | 2746522 | 2746669 | b | 19,79 | M | 4378898 | 4379022 | a |
| 112,08 | M | 2748604 | 2748714 | a | 105,76 | M | 4393690 | 4393852 | b |
| 98,31 | M | 2751648 | 2751801 | b | 87,16 | M | 4440717 | 4440850 | a |
| 122,86 | M | 2753448 | 2753573 | b | 38,32 | M | 4475230 | 4475388 | a |
| 28,86 | M | 2773611 | 2773733 | a | 108,53 | M | 4567298 | 4567417 | b |
| 121,63 | M | 2774815 | 2774946 | b | 125,37 | M | 4573292 | 4573387 | a |
| 28,24 | M | 2778898 | 2779030 | a | 11,06 | M/E | 4646267 | 4646382 | a |
| 128,15 | M | 2782551 | 2782659 | a | 87,2 | M | 4659206 | 4659330 | b |
| 145,7 | M | 2787126 | 2787249 | b | 78,11 | M | 4676295 | 4676448 | a |
| 72,78 | M | 2815439 | 2815583 | a | 83,91 | M | 4754463 | 4754580 | a |
| 20,93 | M | 2830134 | 2830270 | b | 125,26 | M | 4796016 | 4796125 | a |
| 45,38 | M | 2883213 | 2883315 | a | 39,47 | M | p072634 | p072748 | b |
| 119,75 | M | 2929016 | 2929158 | b | 135,29 | M | p084792 | p84902 | b |
| 130,24 | M | 3028011 | 3028128 | a | 134,8 | M | 3065700 | 3065810 | b |
| 53,83 | M | 3036510 | 3036654 | a | 144,32 | M | 3107713 | 3107817 | a |
| 66,12 | M | 3037349 | 3037459 | b | 99,36 | M | 3144890 | 3145017 | a |

| clone number | assay | insertion site a | insertion site b | sequence start |
|---|---|---|---|---|
| 42,65 | M | 3153439 | 3153553 | a |
| 86,28 | M | 3204571 | 3204684 | a |
| 12,65 | M | 3227511 | 3227639 | a |
| 39,47 | M | 3345305 | 3345454 | a |
| 120,8 | M | 3431150 | 3431312 | a |
| 83,64 | M | 3449585 | 3449695 | a |
| 59,79 | M | 3456255 | 3456386 | a |
| 52,06 | M | 3478625 | 3478735 | b |
| 53,84 | M | 3508826 | 3508978 | a |
| 92,01 | M | 3535706 | 3535816 | a |
| 130,13 | M | 3581071 | 3581181 | b |
| 94,51 | M | 3622567 | 3622723 | b |
| 145,87 | M | 3878065 | 3878222 | a |
| 105,32 | M | 3917781 | 3917906 | a |
| 49,8 | M | 3974873 | 3975002 | a |
| 108,28 | M | 4048957 | 4049080 | a |
| 21,9 | M | 4063645 | 4063781 | a |
| 41,41 | M | 4077375 | 4077449 | b |
| 107,73 | M | 4079955 | 4080068 | b |
| 103,44 | M | 4128884 | 4129001 | a |
| 77,42 | M | 4179975 | 4180135 | b |
| 21,01 | M | 4184413 | 4184542 | a |
| 140,28 | M | 4207550 | 4207685 | b |
| 89,78 | M | 4213822 | 4213990 | a |
| 76,68 | M | 4216914 | 4217036 | b |
| 19,71 | M | 4271408 | 4271511 | a |
| 102,88 | M | 4273317 | 4273437 | b |
| 12,57 | E | 4300668 | 4300791 | a |
| 126,32 | M | 4341769 | 4341884 | a |
| 62,12 | M | 4370446 | 4370575 | b |

**Claims**

1. Method for the identification of microbial nucleic acid sequences which are essential for infectivity and/or intracellular survival and/or propagation in eukaryotic host cells comprising the steps

(a) subjecting microbial cells to genome saturating mutagenesis comprising the steps:

(a1) cloning of a genome-representing fragment library of a microbial organism into a conditionally replicating vector, suitable for insertional duplication mutagenesis,
(a2) transforming the vector comprising the fragment library of

(a1) into the microbial organism of (a1) under permissive conditions, and

(a3) producing mutant cells by insertional duplication mutagenesis,

(b) cultivating the mutant cells of step (a) and obtaining a library of viable mutant cells,
(c) contacting permissive eukaryotic host cells with a single clone of the library of step (b) under conditions, wherein an intracellular uptake of the microbial cells into the eukaryotic host cells can take place,
(d) removing non-intracellular microbial cells from the eukaryotic host cells of step (c) and
(e) identifying the microbial cells having a reduced capacity of infectivity and/or intracellular survival and/or propagation in said eukaryotic host cells and
(f) characterizing the obtained nucleic acid sequences and/or polypeptides encoded thereby associated with said reduced capacity,

2. The method of claim 1, wherein said microbial cells are selected from Gram-negative and Gram-positive bacteria, particularly Enterobacteriaceae, Pseudomonadaceae, Vibrionaceae, Neisseriaceae, Chlamydiaceae, Micrococcaceae, Mycobacteriaceae, Pasteurellaceae, Clostridium or Spirochaetales, and more particularly Salmonella spec., Pseudomonas aeruginosa, Vibrio cholerae, Neisseria gonorrhoeae, Chlamydia trachomatis, Streptococcus pneumoniae, Haemophilus influenzae or Leptospira interrogans.

3. The method of claim 1 or 2, wherein said permissive eukaryotic host cells are mammalian cells, particularly human or mouse cells, e.g. selected from macrophages, epithelial cells, fibroblasts, endothelial cells, dendritic cells or muscle cells.

4. The method of any one of the previous claims, wherein between 1 to 100 clonal microbial cells of step (b) are used per permissive eukaryotic host cell in step (c), particularly about 50 microbial cells per epithelial cell or about 10 microbial cells per macrophage.

5. The method of any one of the previous claims, wherein contacting said permissive eukaryotic host cells in step (c) takes between 15 min and 2 hours, particularly about 30 min for macrophages or about 1 hour for epithelial cells.

6. The method of any one of the previous claims, wherein removing of the microbial cells in step (d) is performed by washing the eukaryotic host cells, particularly with physiological buffer or medium and/or incubating the eukaryotic host cells with an antimicrobial agent.

7. The method of claim 6, wherein incubating the eukaryotic host cells with an antimicrobial agent in step (d) takes place between 5 min and 24 hours, preferably about 15 min for epithelial cells or about 1 5 hours for macrophages.

8. The method of any one of the previous claims, wherein identifying the microbial cells in step (e) comprises incubating the eukaryotic host cells under conditions, wherein propagation of intracellular microbial cells can take place and subsequently determining the presence and/or amount of microbial cells within the eukaryotic host cells.

9. The method of claim 8, wherein the determining step comprises lysing the eukaryotic host cells of step (d) under conditions permitting the survival of the microbial cells within the eukaryotic host cells and thereby obtaining viable microbial cells.

10. The method of claim 9 further comprising determining the growth of said viable microbial cells and comparing said growth to the growth of control cells.

11. The method of claim 10, wherein the growth is determined by monitoring the optical density of said microbial cells in a non-static growth phase, particularly in an exponential growth phase.

12. The method of any one of the previous claims, wherein characterizing the nucleic acid sequences and/or polypeptides encoded thereby in step (f) comprises comparative genomics.

13. The method of claim 12, wherein comparative genomics comprises the identification of orthologs.

14. Use of the method of any one of the previous claims for the identification and/or priorization of attenuated microbial cells, particularly bacteria.

15. Use of the method of any one of claims 1 to 13 for the identification and/or priorization of drug targets in microbial cells, particularly bacteria.

**Patentansprüche**

1. Verfahren zur Identifizierung mikrobieller Nukleinsäuresequenzen, welche für Infektivität und/oder intrazelluläres Überleben und/oder Vermehrung in eukaryotischen Wirtszellen essentiell sind, umfassend die Schritte

   (a) Unterziehen von mikrobiellen Zellen einer genomsättigenden Mutagenese umfassend die Schritte:

   (a1) Klonieren einer genomrepräsentierenden Fragmentbibliothek eines mikrobiellen Organismus' in einen konditionell replizierenden Vektor, welcher für eine insertionale Duplikationsmutagenese geeignet ist,
   (a2) Transformieren des Vektors umfassend die Fragmentbibliothek aus (a1) in den mikrobiellen Organismus aus (a1) unter permissiven Bedingungen, und
   (a3) Herstellen von Mutantenzellen durch insertionale Duplikationsmutagenese,

   (b) Kultivieren der Mutantenzellen aus Schritt (a) und Erhalten einer Bibliothek lebensfähiger Mutantenzellen,
   (c) Inkontaktbringen permissiver eukaryotischer Wirtszellen mit einem einzelnen Klon der Bibliothek aus Schritt (b) unter Bedingungen, unter denen eine intrazelluläre Aufnahme der mikrobiellen Zellen in die eukaryotischen Wirtszellen stattfinden kann,
   (d) Entfernen nichtintrazellulärer mikrobieller Zellen von den eukaryotischen Wirtszellen aus Schritt (c) und
   (e) Identifizieren der mikrobiellen Zellen, welche eine verminderte Kapazität zur Infektivität und/oder zum intrazellulären Überleben und/oder zur Vermehrung in den eukaryotischen Wirtszellen haben und
   (f) Charakterisieren der erhaltenen Nukleinsäuresequenzen und/oder hierdurch kodierten Polypeptide, welche mit der verminderten Kapazität assoziiert sind.

2. Verfahren gemäß Anspruch 1, wobei die mikrobiellen Zellen ausgewählt werden aus gramnegativen und grampositiven Bakterien, insbesondere Enterobacteriaceae, Pseudomonadaceae, Vibrionaceae, Neisseriaceae, Chlamydiaceae, Micrococcaceae, Mycobacteriaceae, Pasteurellaceae, Clostridium oder Spirochaetales und stärker bevorzugt Salmonella spec., Pseudomonas aeruginosa, Vibrio cholerae, Neisseria gonorrhoeae, Chlamydia trachomatis, Streptococcus pneumoniae, Haemophilus influenzae oder Leptospira interrogans.

3. Verfahren gemäß Anspruch 1 oder 2, wobei die permissiven eukaryotischen Wirtszellen Säugerzellen sind, insbesondere menschliche Zellen oder Mauszellen, z. B. ausgewählt aus Makrophagen, Epithelzellen, Fibroblasten, Endothelzellen, dendritischen Zellen oder Muskelzellen.

4. Verfahren gemäß einem der vorhergehenden Ansprüche, wobei zwischen 1 bis 100 klonale mikrobielle Zellen aus Schritt (b) bezogen auf eine permissive eukaryotische Wirtszelle in Schritt (c) verwendet werden, insbesondere etwa 50 mikrobielle Zellen bezogen auf eine Epithelzelle oder etwa 10 mikrobielle Zellen bezogen auf einen Makrophagen.

5. Verfahren gemäß einem der vorhergehenden Ansprüche, wobei Inkontaktbringen der permissiven eukaryotischen Wirtszellen in Schritt (c) zwischen 15 min und 2 Stunden dauert, insbesondere etwa 30 min für Makrophagen oder etwa 1 Stunde für Epithelzellen.

6. Verfahren gemäß einem der vorhergehenden Ansprüche, wobei Entfernen der mikrobiellen Zellen in Schritt (d) durch Waschen der eukaryotischen Wirtszellen durchgeführt wird, insbesondere mit physiologischem Puffer oder Medium und/oder Inkubieren der eukaryotischen Wirtszellen mit einem antimikrobiellen Mittel.

7. Verfahren gemäß Anspruch 6, wobei Inkubieren der eukaryotischen Wirtszellen mit einem antimikrobiellen Mittel in Schritt (d) zwischen 5 min und 24 Stunden dauert, vorzugsweise etwa 15 min für Epithelzellen oder etwa 15 Stunden für Makrophagen.

8. Verfahren gemäß einem der vorhergehenden Ansprüche, wobei Identifizieren der mikrobiellen Zellen in Schritt (e) umfasst Inkubieren der eukaryotischen Wirtszellen unter Bedingungen, unter denen Vermehrung intrazellulärer mikrobieller Zellen stattfinden kann, und danach Bestimmen der Anwesenheit und/oder Menge mikrobieller Zellen innerhalb der eukaryotischen Wirtszellen.

9. Verfahren gemäß Anspruch 8, wobei der Bestimmungsschritt umfasst Lysieren der eukaryotischen Wirtszellen aus Schritt (d) unter Bedingungen, welche das Überleben der mikrobiellen Zellen innerhalb der eukaryotischen Wirtszellen erlauben, und hierdurch Erhalten lebensfähiger mikrobieller Zellen.

10. Verfahren gemäß Anspruch 9 weiterhin umfassend Bestimmen des Wachstums der lebensfähigen mikrobiellen Zellen und Vergleichen des Wachstums mit dem Wachstum von Kontrollzellen.

11. Verfahren gemäß Anspruch 10, wobei das Wachstum durch Überwachen der optischen Dichte der mikrobiellen Zellen in einer nichtstatischen Wachstumsphase bestimmt wird, insbesondere in einer exponentiellen Wachstumsphase.

12. Verfahren gemäß einem der vorhergehenden Ansprüche, wobei Charakterisieren der Nukleinsäuresequenzen und/oder hierdurch kodierten Polypeptide in Schritt (f) vergleichende Genomik umfasst.

13. Verfahren gemäß Anspruch 12, wobei die vergleichende Genomik die Identifizierung von Orthologen umfasst.

14. Verwendung des Verfahrens gemäß einem der vorhergehenden Ansprüche zur Identifizierung und/oder Priorisierung von attenuierten mikrobiellen Zellen, insbesondere Bakterien.

15. Verwendung des Verfahrens gemäß einem der Ansprüche 1 bis 13 für die Identifizierung und/oder Priorisierung von Wirkstoffzielen in mikrobiellen Zellen, insbesondere Bakterien.

**Revendications**

1. Procédé d'identification de séquences d'acides nucléiques microbiennes qui sont essentielles pour l'infectivité et/ou la survie intracellulaire et/ou la propagation dans des cellules hôtes eucaryotes comprenant les étapes consistant à :

    (a) soumettre des cellules microbiennes à une mutagenèse par saturation du génome comprenant les étapes consistant à :

      (a1) cloner une bibliothèque de fragments représentant un génome d'un organisme microbien dans un vecteur à réplication conditionnelle, approprié pour une mutagenèse insertionnelle par duplication,
      (a2) transformer le vecteur comprenant la bibliothèque de fragments de (a1) dans l'organisme microbien de (a1) dans des conditions permissives, et
      (a3) produire des cellules mutantes par mutagenèse insertionnelle par duplication,

    (b) cultiver les cellules mutantes de l'étape (a) et obtenir une bibliothèque de cellules mutantes viables,
    (c) mettre en contact les cellules hôtes eucaryotes permissives avec un unique clone de la bibliothèque de l'étape (b) dans des conditions où une absorption intracellulaire des cellules microbiennes dans les cellules hôtes eucaryotes peut avoir lieu,
    (d) éliminer les cellules microbiennes non-intracellulaires des cellules hôtes eucaryotes de l'étape (c) et
    (e) identifier les cellules microbiennes possédant une capacité réduite d'infectivité et/ou de survie intracellulaire et/ou de propagation dans lesdites cellules hôtes eucaryotes et
    (f) caractériser les séquences d'acides nucléiques obtenues et/ou les polypeptides ainsi codés associés à ladite capacité réduite.

2. Procédé selon la revendication 1, dans lequel lesdites cellules microbiennes sont choisies parmi des bactéries Gram-positif et Gram-négatif, en particulier les Enterobacteriaceae, les Pseudomonadaceae, les Vibrionaceae, les Neisseriaceae, les Chlamydiaceae, les Micrococcaceae, les Mycobacteriaceae, les Pasteurellaceae, les Clostridium ou les Spirochaetales, et plus particulièrement Salmonella spec., Pseudomonas aeruginosa, Vibrio cholerae, Neisseria gonorrhoeae, Chlamydia trachomatis, Streptococcus pneumoniae, Haemophilus influenza ou Leptospira interrogans.

**3.** Procédé selon la revendication 1 ou 2, dans lequel lesdites cellules hôtes eucaryotes permissives sont des cellules mammifères, en particulier des cellules humaines ou de souris, par exemple choisies parmi les macrophages, les cellules épithéliales, les fibroblastes, les cellules endothéliales, les cellules dendritiques ou les cellules musculaires.

**4.** Procédé selon l'une quelconque des revendications précédentes, dans lequel entre 1 à 100 cellules microbiennes clonales de l'étape (b) sont utilisées par cellule eucaryote hôte permissive dans l'étape (c), en particulier environ 50 cellules microbiennes par cellule épithéliale ou environ 10 cellules microbiennes par macrophage.

**5.** Procédé selon l'une quelconque des revendications précédentes, dans lequel la mise en contact desdites cellules hôtes eucaryotes permissives dans l'étape (c) prend entre 15 minutes et 2 heures, en particulier environ 30 minutes pour des macrophages ou environ 1 heure pour des cellules épithéliales.

**6.** Procédé selon l'une quelconque des revendications précédentes, dans lequel l'élimination des cellules microbiennes dans l'étape (d) est réalisée en lavant les cellules hôtes eucaryotes, en particulier avec un milieu ou un tampon physiologique et/ou en incubant les cellules hôtes eucaryotes avec un agent antimicrobien.

**7.** Procédé selon la revendication 6, dans lequel l'incubation des cellules hôtes eucaryotes avec un agent antimicrobien dans l'étape (d) a lieu entre 5 minutes et 24 heures, de préférence environ 15 minutes pour des cellules épithéliales ou environ 15 heures pour des macrophages.

**8.** Procédé selon l'une quelconque des revendications précédentes, dans lequel l'identification des cellules microbiennes dans l'étape (e) comprend l'incubation des cellules hôtes eucaryotes dans des conditions où la propagation des cellules microbiennes intracellulaires peut avoir lieu puis la détermination de la présence et/ou de la quantité de cellules microbiennes à l'intérieur des cellules hôtes eucaryotes.

**9.** Procédé selon la revendication 8, dans lequel l'étape de détermination comprend la lyse des cellules hôtes eucaryotes de l'étape (d) dans des conditions permettant la survie des cellules microbiennes à l'intérieur des cellules hôtes eucaryotes et donc l'obtention de cellules microbiennes viables.

**10.** Procédé selon la revendication 9, comprenant en outre la détermination de la croissance desdites cellules microbiennes viables et la comparaison de ladite croissance à la croissance de cellules témoins.

**11.** Procédé selon la revendication 10, dans lequel la croissance est déterminée en contrôlant la densité optique desdites cellules microbiennes en phase de croissance non statique, en particulier en phase de croissance exponentielle.

**12.** Procédé selon l'une quelconque des revendications précédentes, dans lequel la caractérisation des séquences d'acides nucléiques et/ou des polypeptides ainsi codés dans l'étape (f) comprend une génomique comparative.

**13.** Procédé selon la revendication 12, dans lequel la génomique comparative comprend l'identification d'orthologues.

**14.** Utilisation du procédé selon l'une quelconque des revendications précédentes pour l'identification et/ou la hiérarchisation de cellules microbiennes atténuées, en particulier des bactéries.

**15.** Utilisation du procédé selon l'une quelconque des revendications 1 à 13 pour l'identification et/ou la hiérarchisation de cibles médicamenteuses dans des cellules microbiennes, en particulier des bactéries.

# Figure 1: Screening method for the identification of facultatively essential microbial genes

Genome saturating mutagenesis of microbial cells

Seeding of permissive eukaryotic host cells

Growth of mutants with an insertion in a non obligatively essential nucleic acid sequence

Infection of permissive eukaryotic host cells with a defined number of microbial cells

Removing non-intracellular microbial cells from the eukaryotic host cells

Propagation of intracellularly growing microbial cells

Lysis of eukaryotic host cells

Propagation of recovered microbial cells in the lysate

Identification and characterization of microbial cells with an insertion in facultatively essential nucleic acid sequences

# Figure 2: Genome saturating mutagenesis

Step a: Fragment library from *Salmonella enterica* serovar Typhimurium in ts-vector is transformed in host strain EC101, growth at 30°C

↓

Step b: Preparation of plasmid-DNA and transformation in *Salmonella enterica* serovar Typhimurium, growth at 30°C

↓

Step c: Insertional duplication mutagenesis by homologous recombination

```
● 0 0 ● 0 0 0 0 0 0 0 0
0 0 ● 0 0 0 0 0 0 0 0 0
0 0 0 0 0 0 ● 0 ● ● 0 0
0 0 0 0 0 0 0 0 0 0 ● 0
0 ● 0 0 0 0 0 ● 0 0 0 0
0 0 ● 0 0 0 0 0 0 0 0 0
0 0 0 0 ● ● 0 0 0 ● 0 0
0 0 0 0 ● 0 0 0 0 0 0 ●
```

● =Clone without insert or fragment from obligatively essential gene

↓

Step d: Growth of all integrants at 38.5°C temperature; clones with insertless vector or obligatively essential gene fragment do not grow

```
0 0    0 0 0 0 0 0 0 0
0 0   0 0 0 0 0 0 0 0
0 0 0 0 0 0   0     0 0
0 0 0 0 0 0 0 0 0 0   0
0   0 0 0 0 0   0 0 0 0
0 0   0 0 0 0 0 0 0 0 0
0 0 0 0      0 0 0   0 0
0 0 0 0   0 0 0 0 0 0
```

↓

Step e: Selection of viable mutant cells

**Figure 3: Plasmids for insertional duplication mutagenesis.**
pIDM shows a plasmid harbouring the origin of replication, a
selectable marker, a multiple cloning site and a replication
protein. A conditionally replicating vector must not provide aν
essential replication protein which might be chromosomally
encoded (see description).

origin of replication

replication
protein

3500

500

3000— pIDM —1000

2500

2000

1500

selectable
marker

multiple cloning site

## Figure 4: Insertional mutagenesis by homologous recombination.

An intragenic fragment of ~400 bp is cloned into the vector pIDM. A cointegrate structure is formed by homologous recombination resulting in fragment duplication and interruption of the targeted nucleic acid sequence. Integrants can be selected by appropriate growth conditions, and the resulting phenotype characterized.